# EUROPÄISCHE PATENTANMELDUNG

(11) **EP 2 060 634 A1**
(43) Veröffentlichungstag der Anmeldung: **20.05.2009**
(21) Anmeldenummer: 08022398.5
(22) Anmeldetag: 13.07.2000
(51) Int. Cl.: C12N 15/864, C12N 15/35, C12N 15/62, C07K 14/015, C12N 5/10, A61K 39/23, A61K 48/00

(54) **Strukturprotein von Adeno-assoziiertem Virus mit veränderter Antigenität, seine Herstellung und Verwendung**

(30) Priorität: 15.07.1999 DE 19933288
(62) Teilanmeldung aus: 00954493.3
(71) Anmelder: MediGene Aktiengesellschaft, 82152 Planegg (DE)
(72) Erfinder: Hallek, Michael, 50935 Köln (DE); Girod, Anne, 82287 Jesenwang (DE); Ried, Martin, 82319 Starnberg (DE)
(74) Vertreter: Bösl, Raphael Konrad

(57) **Zusammenfassung**

Die vorliegende Erfindung betrifft ein Strukturprotein von Adeno-assoziiertem Virus (AAV), das mindestens eine Modifikation enthält, die eine Verringerung der Antigenität bewirkt, seine Herstellung und Verwendung.

SEQUENZPROTOKOLL

<110> MediGene Aktiengesellschaft

<120> Strukturprotein von Adeno-assoziiertem Virus mit veränderter
Antigenität, seine Herstellung und Verwendung

<140> 199 33 288.6

<141> 15.07.1999

<160> 9

<170> Word 6.0, PC-DOS/MS-DOS

<210> 1
<211> 14
<212> PRT
<213> Mus musculus

<400> 1

Gln Ala Gly Thr Phe Ala Leu Arg Gly Asp Asn Pro Gln Gly 14

<210> 2
<211> 10
<212> PRT
<213> Adeno-assoziierter Virus

<400> 2

Tyr Lys Gln Ile Ser Ser Gln Ser Gly Ala 10

<210> 3

<400> 3

Tyr Leu Thr Leu Asn Asn Gly Ser Gln Ala 10

<210> 4
<211> 10

<212> PRT

<213> Adeno-assoziierter Virus

<400> 4

Tyr Tyr Leu Ser Arg Thr Asn Thr Pro Ser 10

<210> 5
<211> 10

<212> PRT

<213> Adeno-assoziierter Virus

<400> 5

Glu Glu Lys Phe Phe Pro Gln Ser Gly Val 10

<210> 6

<211> 5

<212> PRT

<213> Adeno-assoziierter Virus

<400> 6

Asn Pro Val Ala Thr 5

<210> 7

<211> 5

<212> PRT

<213> Adeno-assoziierter Virus

<400> 7

Glu Gln Tyr Gly Ser 5

<210> 8
<211> 10
<212> PRT
<213> Adeno-assoziierter Virus

<400> 8

Leu Gln Arg Gly Asn Arg Gln Ala Ala Thr 10

<210> 9
<211> 10
<212> PRT
<213> Adeno-assoziierter Virus

<400> 9

Asn Val Asp Phe Thr Val Asp Thr Asn Gly 10

## Beschreibung

Die vorliegende Erfindung betrifft ein Strukturprotein von Adeno-assoziiertem Virus (AAV), das mindestens eine Modifikation enthält, die eine Verringerung der Antigenität bewirkt, seine Herstellung und Verwendung.

Das AAV-Virus gehört zur Familie der Parvoviren. Diese zeichnen sich durch ein ikosaedrisches, unbehülltes Kapsid mit einem Durchmesser von 18 bis 30 nm aus, welches eine lineare, einzelsträngige DNA von ca. 5 kb enthält. Für eine effiziente Vermehrung von AAV ist eine Coinfektion der Wirtszelle mit Helferviren, beispielsweise mit Adenoviren, Herpesviren oder Vacciniaviren erforderlich. In Abwesenheit eines Helfervirus geht AAV in einen Latenzzustand über, wobei das Virusgenom in der Lage ist, stabil in das Wirtszellgenom zu integrieren. Die Eigenschaft von AAV, in das Wirtsgenom zu integrieren, macht es als Transduktionsvektor für Säugetierzellen besonders interessant. Für die Vektorfunktionen genügen im allgemeinen die beiden ca. 145 bp langen invertierten terminalen Wiederholungssequenzen (ITR: "Inverted Terminal Repeats"). Sie tragen die "cis" notwendigen Signale für Replikation, Verpackung und Integration in das Wirtszellgenom. Zur Verpackung in rekombinante Vektorpartikel wird ein Helferplasmid, welches die Gene für nicht-strukturelle Proteine (Rep-Proteine) und für strukturelle Proteine (Cap-Proteine) trägt, in verpackungsgeeignete Zellen, z.B. HeLa- oder 293-Zellen, transfiziert, die hierauf beispielsweise mit Adenovirus infiziert werden. Nach einigen Tagen erhält man ein Lysat, welches rekombinante AAV-Partikel enthält. Geeignete Helferplasmide sind z.B. bei Chiorini et al., (1995) Hum. Gene Ther. 6, 1531-1541 oder Girod et al. (1999), Nat. Med. beschrieben.

Das AAV-Kapsid besteht aus drei verschiedenen Proteinen: VP1, VP2 und VP3, deren relative Anteile 5% VP1, 5% VP2 und 90% VP3 sind. Die AAV-Kapsidgene sind am rechten Ende des AAV-Genoms lokalisiert und werden durch überlappende Sequenzen desselben offenen Leserahmens (ORF) unter Verwendung verschiedener Startkodons sowie zweier unterschiedlich gespleißter Varianten eines Transkripts kodiert. Das VP1-Gen enthält die ganze VP2-Gensequenz, welche wiederum die ganze VP3-Gensequenz mit einem spezifischen N-terminalen Bereich enthält. Die Tatsache, daß die überlappenden Leserahmen für alle drei AAV-Kapsid-Proteine kodieren, ist für die obligatorische Expression aller Kapsid-Proteine, wenn auch zu unterschiedlichen Anteilen, verantwortlich.

Die Molekularmassen der Kapsid-Proteine sind 87 kD für VP1, 73 kD für VP2 und 62 kD für VP3. Die Sequenzen der Kapsidgene sind in Srivastava, A. et al. (1983), J. Virol., 45, 555-564; Muzyczka, N. (1992), Curr. Top. Micro. Immunol., 158, 97-129, Ruffing, N. et al. (1992), J. Virol., 66, 6922-6930 oder Rutledge, E. A. et al. (1998) J. Virol. 72, 309-319 beispielsweise beschrieben. Die physikalische und genetische Karte des AAV-Genoms ist beispielsweise bei Kotin, R. M. (1994), Human Gene Therapy, 5, 793-801 beschrieben.

Zudem sind verschiedene AAV-Serotypen bekannt, von denen der menschliche AAV-Serotyp 2 (AAV2) am besten untersucht ist. In diesen Analysen zeigte sich, daß AAV Viren als virale Vektoren vorteilhafte Eigenschaften für die somatische Gentherapie besitzen. Die wesentlichen Vorteile sind die fehlende Pathogenität für den Menschen, die stabile Integration viraler DNA in das zelluläre Genom, die Fähigkeit, nicht teilende Zellen zu infizieren, die Stabilität des Virions, was die Aufreinigung zu hohen Titern (10¹³ bis 10¹⁴ Partikel pro ml) ermöglicht, die relativ geringe Immunogenität sowie das Fehlen viraler Gene und Genprodukte im rekombinanten AAV-Vektor, was unter Sicherheitsaspekten für die Verwendung in der Gentherapie vorteilhaft ist. Die Klonierung von Genen in den AAV-Vektor erfolgt mittlerweile nach den dem Fachmann allgemein bekannten Methoden, wie sie z.B. in WO 95/ 23 867, bei Chiorini, J. A. et al. (1995), Human Gene Therapy, 6, 1531-1541 oder bei Kotin, R. M. (1994), supra, beschrieben sind.

Der Einsatz gerade viraler Vektoren in der Gentherapie ist im hohen Maße von der Antigenität des verwendeten Systems abhängig, da mit einer hohen Antigenität auch eine verstärkte Immunantwort einhergeht, die mit dem Therapieerfolg interferieren könnte. Daher ist auch die Antigenität des AAV-Virus von entscheidender Bedeutung für dessen Verwendbarkeit in der Therapie. Unter dem Begriff Antigen versteht man Stoffe, die nach Einführung in den Organismus von Menschen und Tieren eine spezifische Immunantwort auslösen. Diese äußert sich entweder in der Bildung von Antikörpern (humorale Immunantwort) und der Entwicklung einer zellvermittelten Immunität (zelluläre Immunantwort) oder einer spezifischen immunologischen Toleranz. Voraussetzung einer Immunantwort (für die Immunogenität des Antigens) ist im allgemeinen, daß das Antigen vom Organismus als fremd erkannt wird, daß es ein MW > 1 kDa besitzt und der Stoffklasse der Proteine oder Polysaccharide, seltener Desoxyribonucleinsäuren oder Lipide angehört. Komplexere Strukturen wie z.B. Bakterien, Viren oder Erythrocyten (partikuläre Antigene) sind im allgemeinen noch wirksamere Antigene, besitzen also hohe Antigenität. Unter Antigenität versteht man daher im Sinne dieser Erfindung die Fähigkeit mit dem Immunsystem (humorales und zelluläres) durch Bindung zu interagieren (erkannt zu werden). Der Begriff umfaßt dabei auch die Immunogenität, also auch die Fähigkeit zur Auslösung einer Immunantwort. Gerade bei Viren können dabei prinzipiell antigene Strukturen für die Antikörperbindung nicht nur durch die Primärstruktur, sondern auch durch die Sekundär-, Tertiär- oder Quartärstruktur der Kapsid-Proteine bzw. Kapside bestimmt sein.

Chapman M. S. und Rossmann M. G. (1993), Virology, 194, 491-508 konnten durch Sequenzvergleiche mit verschiedenen Parvoviren, aus dem die antigenen Unterschiede zwischen den Kapsidproteinen vorhergesagt wurden, die wichtigsten Antigen-Determinanten des CPV-Kapsids identifizieren. Nach dieser Untersuchung ist die Antigenität des CPV-Kapsid-Proteins primär an extern exponierte Loops mit hoher Sequenz-Variabilität gebunden. Beim AAV-Virus-Kapsid hingegen gibt es derartige Untersuchungen noch nicht. Lediglich die WO 96/00587 beschreibt AAV-Kapsid-Fusionsproteine, bei denen beispielsweise in die für ein Kapsid-Protein codierende DNA die für ein klinisch relevantes Antigen codierende DNA eingefügt wird, ohne daß dies mit der Kapsidbildung interferiert, und das Konstrukt als AAV-Kapsid-Fusionsprotein exprimiert wird. Die klinisch relevanten Antigene sind Epitope, die beispielsweise aus Bakterien (z.B. Salmonella), Viren (z.B. env-HIV) oder Tumorzellen stammen. Die entstehenden AAV-Kapsid-Fusionsproteine sollen eine Immunantwort auslösen, also für eine gesteigerte Antigenität der AAV-Viren sorgen.

Eine verringerte Antigenität des AAV wird im Stand der Technik nicht diskutiert. Für die praktische Anwendung von AAV-Vektoren - gerade in der Gentherapie - ist aber eine verringerte Antigenität im Vergleich zum Wildtyp oder zu vom Wildtyp abgeleiteten AAV-Vektoren von Vorteil. Denn auch Wildtyp-AAV hat durchaus Antigen-Determinanten. So gibt es anti-AAV2 Ig positive Individuen, bei denen eine Therapie mit AAV-Vektoren einer Wildtyp-Antigenität zwangsläufig schwierig bis unmöglich ist. Ebenso könnte ein Patient bei wiederholter Therapie mit AAV-Vektoren zunehmend eine humorale und/oder zelluläre Immunantwort gegen die verwendeten AAV-Vektoren entwickeln. Eine derartige Immunisierung würde den Erfolg einer Therapie schmälern bzw. verhindern. Je geringer also die Antigenität eines rekombinanten AAV-Virus ist oder je mehr sich seine Antigenität von einem Wildtyp-Virus oder einem zuvor verwendeten rekombinanten AAV-Virus unterscheidet, desto erfolgversprechender erscheint dessen therapeutischer Einsatz.

Aufgabe der vorliegenden Erfindung war es daher, die Antigenität des AAV-Virus insbesondere eines Strukturproteins gegenüber dem Wildtyp zu verringern. Insbesondere sollten durch Modifikation AAV-Vektoren entwickelt werden, die einen spezifischen und effizienten Gentransfer ermöglichen, aber der Immunantwort besser oder vollständig entgehen. Daher sollte die Modifikation bevorzugt so erfolgen, daß sich gleichzeitig die Infektiosität des Virus nicht wesentlich verringert oder zumindest erhalten bleibt.

Überraschenderweise wurde nun gefunden, daß Struktur- bzw. Kapsid-Proteine von AAV so modifiziert werden können, daß dadurch eine Verringerung der Antigenität bewirkt wird, wobei sich auch die Infektiosität nicht wesentlich verringert, diese zumindest erhalten bleibt.

Ein Gegenstand der vorliegenden Erfindung ist daher ein Strukturprotein von AAV, das mindestens eine Modifikation enthält, die eine Verringerung der Antigenität bewirkt.

Unter der Verringerung der Antigenität versteht man im Sinne der Erfindung und obiger Definitionen die Verringerung der Antikörperbildung und/oder Antikörperbindung durch Veränderung, Entfernen oder Hinzufügen bestimmter Sequenzen oder Epitope oder einer Kombination dieser Maßnahmen, insbesondere bestimmter im Wildtyp vorhandener Epitope und Sequenzen. Durch eine verminderte Antigenität wird beispielsweise eine Immunisierung eines Organismus durch eine Therapie mit einem AAV-Vektor verringert. Dabei ist auch eine absolut gesehen, d.h. im Durchschnitt der Stärke der Immunantwort, lediglich veränderte Antigenität im Sinne dieser Erfindung als verringert anzusehen, wenn mit dem erfindungsgemäßen Strukturprotein eine Antikörper-(Immun-)Antwort nicht ausgelöst wird, die mit dem Wildtyp ausgelöst worden wäre. Eine derartige, absolut gesehen lediglich veränderte Antigenität kann zu einer verringerten Immunisierung führen, wenn bei aufeinanderfolgenden Therapien erfindungsgemäße AAV-Vektoren mit unterschiedlicher Antigenität eingesetzt werden. Die veränderte Antigenität kann sich dabei sowohl auf die humorale wie auch die zelluläre Immunantwort beziehen.

Die verringerte Antigenität läßt sich für die humorale Immunantwort beispielsweise dadurch nachweisen, daß ein Antikörper, der an das unmodifizierte (Wildtyp-) AAV-Kapsid-Protein oder AAV-Kapsid binden kann, das erfindungsgemäße, modifizierte AAV-Kapsid-Protein oder AAV-Kapside nicht mehr oder wesentlich schlechter erkennt. Derartige Nachweise können durch Standardverfahren wie ein Enzyme linked Immuno-absorbent Assay (ELISA) durchgeführt werden. Ein geeigneter Antikörper ist beispielsweise der A20 monoklonale Antikörper (siehe Wistuba, A. et al. (1997) J. Virol., 71, 1341-52), der spezifisch nur vollständig assemblierte AAV2-Kapside des Wildtyps erkennt, jedoch keine freien Kapsidproteine.

Für die zelluläre Immunantwort kann ein Nachweis der veränderten Antigenität darüber geführt werden, daß AAV-spezifische Immunzellen durch Antigen-präsentierende Zellen, die mit Partikeln aus modifizierten Strukturproteinen infiziert wurden, nicht so stark stimuliert werden wie durch Antigen-präsentierende Zellen, die mit Partikeln aus ursprünglichen Strukturproteinen infiziert wurden. Dieses Verfahren ist in Analogie zu den Verfahren für Vakzinia- und Adenoviren (Tarpey, I. et al., (1994), Immunology, 81, 222-7; Nimako, M. et al., (1997), Cancer Res. 57, 4855-61). Die Stimulation der Immunzellen läßt sich beispielsweise durch einen Cytokinassay (Chapter 6.2 bis 6.24 in Current Protocols in Immunology (1999), edited by Coligan J.E. et al., John Wiley & Sons) quantitativ messen.

Es ist besonders bevorzugt, daß die Modifikation im erfindungsgemäßen Strukturprotein keine wesentliche Verringerung der Infektiosität des Virus bewirkt, bzw. die Infektiosität zumindest erhalten bleibt. Unter Infektiosität versteht man im Sinne-dieser Erfindung die Fähigkeit, Zellen zu transduzieren.

Des weiteren ist das erfindungsgemäße Strukturprotein vorzugsweise weiterhin zur Partikelbildung, d.h. zur Ausbildung eines ikosaedrischen Kapsids, befähigt, insbesondere in Form eines AAV-Kapsids, da Partikel bzw. Kapside als Träger von ausgewählten Verbindungen, z.B. rAAV-Transduktionsvektoren, besonders geeignet sind. Die Bildung von Partikeln läßt sich beispielsweise durch Elektronenmikroskopie nachweisen. Ein anderer Nachweis ist das Sedimentationsverhalten während einer Cäsiumchlorid-Dichtegradientenzentrifugation mit anschließendem, optionalen Nachweis von in den Partikeln enthaltener viraler DNA.

Im allgemeinen kann die Modifikation im VP1-, VP2- und/oder VP3-Strukturprotein liegen, wobei das VP1- und/oder das VP3-Strukturprotein bevorzugt sind. Des weiteren kann das Strukturprotein von allen AAV-Serotypen abgeleitet sein, insbesondere von humanen Serotypen, vorzugsweise von AAV1, AAV2, AAV3, AAV4 AAV5 und/oder AAV6, vor allem von AAV2, AAV3 und/oder AAV6.

Vorzugsweise ist/sind die Modifikation/en an der Virusoberfläche lokalisiert. Zur Bestimmung der oberflächen-lokalisierten Bereiche der Strukturproteine wurde gemäß der vorliegenden Erfindung überraschenderweise gefunden, daß CPV(Canine-Parvovirus) - und AAV2-Sequenzen und -Strukturen vergleichbar sind. Man kann daher vorzugsweise auf bekannte Kristallstrukturen von Parvoviren wie von Parvovirus B19 oder von CPV zurückgreifen und mit Hilfe von Homologievergleichen Proteindomänen identifizieren, die auf der Virusoberfläche lokalisiert sind. Gemäß der vorliegenden Erfindung hat daher beispielsweise ein computerunterstützter Vergleich zwischen CPV und AAV2 bzw. Parvovirus B19 und AAV2 überraschenderweise reproduzierbar zur Identifikation von Schleifen (Loops) in VP3 geführt, deren Sequenz variiert, d.h. die eine geringe Homologie besitzen und die voraussichtlich auf der Virusoberfläche lokalisiert sind. Da die Antigene für die humorale Immunantwort für Antikörper zugänglich und somit auf der Virusoberfläche sein müssen, stellen diese Schleifen bevorzugte Kandidaten für Modifikationen dar. So wurde die bekannte Kristallstruktur des CPV VP2-Kapsid-Proteins (z.B. Luo M.(1988), J. Mol. Biol., 200, 209-211; Wu und Rossmann (1993), J.Mol.Biol., 233, 231-244; Tsao J. et al. (1991) Science, 251, 1456-1464) aufgrund der hohen Ähnlichkeit zum AAV2 VP3 in der sekundären Struktur des Proteins als Muster genommen, um die Regionen herauszufinden, die auf der viralen Kapsidoberfläche exponiert sind und die aufgrund der lokalen Aminosäuresequenz flexibel genug sind, beispielsweise die Insertion -einer Peptidsequenz zu überstehen. Dabei wurde sorgfältig darauf geachtet, daß keine sekundären Strukturelemente des AAV2-Kapsidproteins ausgewählt wurden, die das Kapsid destabilisieren würden.

In einer bevorzugten Ausführungsform sind die Modifikation(en) am N-Terminus des Strukturproteins lokalisiert, da gefunden wurde, daß beispielsweise bei Parvovirus B 19 der N-Terminus an der Zelloberfläche liegt.

Eine weitere Möglichkeit zur Bestimmung der Oberflächen-lokalisierten Bereiche der Strukturproteine ist ein Vergleich der für die Kapside kodierenden Nukleinsäuresequenzen von unterschiedlichen AAV-Serotypen. Hierzu können beispielsweise bekannte DNA-Sequenzen unterschiedlicher AAV-Serotypen, wie AAV1, AAV2, AAV3, AAV4, AAV5 oder AAV6, für Strukturanalysen möglicher Kapsidmorphologien von beispielsweise AAV2 herangezogen werden, wobei ab initio mögliche Tertiärstrukturen berechnet und Sequenzbereiche aufgrund allgemein bekannter Aminosäure-Eigenschaften den inneren oder äußeren Kapsidbereichen zugeordnet werden können. So konnten beispielsweise gemäß der vorliegenden Erfindung mögliche Insertionsstellen im VP3-Bereich des AAV2-Kapsids ermittelt werden, die die Insertion beispielsweise von Peptiden und deren Expression auf der Virusoberfläche ermöglichten (siehe unten).

Unter einer Modifikation wird z. B. eine Veränderung der Kapsidproteine verstanden, die durch kovalente oder nicht-kovalente Bindung eines Moleküls an eine oder mehrerer Aminosäuren oder Aminosäuresequenzen erzielt wird. So kann ein Kapsidprotein z. B. durch kovalente Bindung von Mono- oder Oligosacchariden, Biotin oder anderen hochmolekularen Verbindungen an eine oder mehrere Aminosäuren modifiziert werden. Die Modifikation kann aber auch durch kovalente Bindung niedermolekularer Verbindungen wie einer Hydroxylgruppe an eine oder mehrere Aminosäuren erreicht werden. Ferner können Moleküle oder Molekül-Komplexe über nicht-kovalente Bindung an die Kapsidproteine angelagert werden und so antigene Regionen abschirmen. Dies kann beispielsweise die Antigen-Bindungsstelle von Immunglobulinen sein, z. B. ein F_{ab}-Fragment oder andere Moleküle, die eine hohe Affinität zu der antigenen Region oder benachbarten Regionen aufweisen. Derartige Moleküle können beispielsweise aus Molekül-Banken im Hinblick auf ihre Affinität gescreent werden. Bei bekannter dreidimensionler Struktur der antigenen Region oder des Kapsidproteins können eine Reihe von potentiell bindenden Molekülen konzipiert und synthetisiert werden, die anschließend auf ihre Affinität getestet werden können.

Unter Modifikation wird beispielsweise aber auch eine oder mehrere Mutationen, also Veränderungen der Abfolge der Aminosäuren, verstanden. Der Begriff Mutation beinhaltet z. B. eine Punktmutation, eine Mutation mehrerer Aminosäuren, eine oder mehrere Deletion(en), eine oder mehrere Insertion(en) oder eine Kombination dieser Mutationen sein. Dabei kann die Punktmutation oder die Mutation mehrerer Aminosäuren innerhalb von T- oder B-Zellepitopen liegen und die Modifikation gleichzeitig aus Punktmutationen, Mutationen mehrerer Aminosäuren, Insertionen und/oder Deletionen bestehen.

In einer bevorzugten Ausführung wird Protein oder Peptid, vorzugsweise immunsuppressives Protein oder Peptid, insertiert. Dabei kann das Peptid aus beispielsweise 5 bis 30 Aminosäuren, vorzugsweise 8 bis 20 Aminosäuren und insbesondere 10 bis 18 Aminosäuren bestehen. Das Peptid hat beispielsweise die Sequenz QAGTFALRGDNPQG oder eine Sequenz, die zu dieser stark homolog ist.

Besonders bevorzugt ist ein erfindungsgemäßes Strukturprotein, das mindestens eine weitere Modifikation enthält. Darunter ist zu verstehen, daß das Strukturprotein neben einer Modifikation, die eine Verringerung der Antigenität des Virus bewirkt, auch eine weitere Modifikation enthält, die nicht zwangsläufig auch eine Verringerung der Antigenität des Virus bewirkt. Besonders bevorzugt ist hier eine weitere Modifikation, die eine Änderung, vorzugsweise Erhöhung, der Infektiosität des Virus bewirkt.

In einer weiteren bevorzugten Ausführungsform stellt/stellen die weiteren Modifikation/en eine oder mehrere Deletionen und/oder eine oder mehrere Insertionen im Strukturprotein oder Kombinationen dieser Modifikationen dar. Dabei ist die Insertion vorzugsweise die Insertion eines Zellmembranrezeptor-Liganden, eines Rep-Proteins oder -Peptids, beispielsweise in Form einer Rep-Domäne, eines immunsuppressiven Proteins oder Peptids und/oder eines Proteins oder Peptids mit einem Signal zur Doppelstrangsynthese eines Transgens bzw. Fremdgens.

Beispiele von weiteren Insertionen sind u.a. Integrine, Cytokine oder Rezeptor-Bindungsdomänen von Cytokinen, Integrinen oder Wachstumsfaktoren, wie z.B. GM-CSF, IL-2, IL-12, CD40L, TNF, NGF, PDGF oder EGF, an Zelloberflächenrezeptoren bindende einzelkettige Antikörper, sog. "single chain" Antikörper (scFv), beispielsweise an die Oberflächenrezeptoren CD40, CD40L, B7, CD28 oder CD34 bindende einzelkettige Antikörper, oder Epitope bzw. Rezeptorbindungsstellen, die beispielsweise ihrerseits von bestimmten Antikörpern, beispielsweise Anti-CD40L-monoklonale Antikörper, bzw. von chemischen Substanzen oder Hormonen, z.B. Katecholamine, erkannt werden.

In einer bevorzugten Ausführungsform der weiteren Modifikation werden antikörperbindende Strukturen, wie z.B. Protein A, Protein G oder anti-Fc-Antikörper, bzw. Teile hiervon, insertiert. An diese werden wiederum spezifische Antikörper gegen bestimmte Zelloberflächenstrukturen (beispielsweise gegen das CD40 bei lymphatischen Zellen oder gegen das CD34 bei hämatopoietische Zellen) angekoppelt.

In einer bevorzugten Ausführungsform wird (werden) die Modifikation(en) durch eine oder mehrere Insertionen an der XhoI-Schnittstelle der VP1-kodierenden Nukleinsäure und in einer anderen bevorzugten Ausführungsform an der BsrBI-Schnittstelle der VP1-kodierenden Nukleinsäure bewirkt. Eine weitere bevorzugte Ausführungsform des erfindungsgemäßen Strukturproteins entsteht durch eine Deletion zwischen den BsrBI-HindII-Schnittstellen der VP1-kodierenden Nukleinsäure und eine oder mehrere Insertionen, vorzugsweise an der Stelle der Deletion.

In einer weiteren bevorzugten Ausführungsform der vorliegenden Erfindung wird (werden) die Modifikation(en) durch eine oder mehrere Deletionen zwischen den XhoI-XhoI-Schnittstellen der VP1-kodierenden Nukleinsäure, die 62 Aminosäuren umfaßt (Hermonat, P. L. et al. (1984), J. Virol., 51, 329-339) bewirkt. In einer weiteren bevorzugten und entsprechenden Ausführungsform liegt die Deletion(en) zwischen den BsrBI-HindII-Schnittstellen der VP1-kodierenden Nukleinsäure, die innerhalb der oben beschriebenen Deletion liegt und 29 Aminosäuren umfaßt. Diese Deletion hat den Vorteil, daß sie keine Überlappung mit dem rep-Gen hat und daher den Verpackungsmechanismus im wesentlichen nicht beeinflußt.

In einer weiteren bevorzugten Ausführungsform liegen ein oder mehrere Insertionen im VP3-Strukturprotein (Rutledge, E. A. et al. (1998) supra) vor und/oder nach mindestens einer Aminosäure in der Sequenz ausgewählt aus YKQIS SQSGA, YLTLN NGSQA, YYLSR TNTPS, EEKFF PQSGV, NPVAT, EQYGS, LQRGN RQAAT, NVDFT VDTNG, da diese Stellen an den exponierten Stellen eines Loops liegen, wobei das Risiko gering ist, die VP3-Struktur zu ändern.

Die Punktmutation(en), die Mutation(en) mehrerer Aminosäuren, die Deletion(en) oder Insertion(en) wird/werden nach allgemein bekannten Methoden durch Deletion und Insertion in dem für das Strukturprotein codierenden Gen durchgeführt. Die Deletionen lassen sich beispielsweise mittels PCR-unterstützter Mutagenese in die einzelnen Strukturprotein-Gene einführen. Die Insertionen lassen sich nach allgemein bekannten Methoden beispielsweise mittels Hydrolyse durch Restriktionsendonukleasen der entsprechenden Strukturprotein-Gene und anschließender Ligasereaktion einfügen. Die anschließende Expression des mutierten Gens führt zum erfindungsgemäßen Strukturprotein.

Ein anderer Gegenstand der vorliegenden Erfindung ist auch ein erfindungsgemäßes Strukturprotein in Form eines AAV-Partikels, insbesondere in Form eines AAV-Kapsids, da Partikel bzw. Kapside als Träger von ausgewählten Verbindungen, z.B. rAAV-Transduktionsvektoren, besonders geeignet sind.

Weitere Gegenstände der vorliegenden Erfindung sind eine Nukleinsäure, vorzugsweise eine RNA oder DNA, insbesondere eine doppelsträngige DNA, kodierend für ein erfindungsgemäßes Strukturprotein.

Die vorliegende Erfindung bezieht sich auch auf eine Zelle, vorzugsweise eine Säugetierzelle, beispielsweise eine COS-Zelle, HeLa-Zelle oder 293-Zelle, enthaltend eine erfindungsgemäße Nukleinsäure. Derartige Zellen eignen sich beispielsweise zur Herstellung der rekombinanten AAV-Partikel.

Ein weiterer Gegenstand der vorliegenden Erfindung ist daher auch ein Verfahren zur Herstellung eines erfindungsgemäßen Strukturproteins, insbesondere zur Herstellung eines erfindungsgemäßen Strukturproteins in Form eines AAV-Partikels, wobei eine geeignete Zelle, enthaltend eine Nukleinsäure, kodierend für das erfindungsgemäße Strukturprotein kultiviert und ggf. das exprimierte Strukturprotein isoliert wird. Beispielsweise läßt sich das erfindungsgemäße Strukturprotein über einen Cäsiumchlorid-Gradienten, wie beispielsweise in Chiorini, J. A. et al. (1995), supra, beschrieben, isolieren.

Ein anderer Gegenstand der vorliegenden Erfindung bezieht sich auch auf ein Arzneimittel, enthaltend ein erfindungsgemäßes Strukturprotein oder eine erfindungsgemäße Nukleinsäure oder eine erfindungsgemäße Zelle und ggf. geeignete Hilfs- und Zusatzstoffe, wie z.B. eine physiologische Kochsalzlösung, Stabilisatoren, Proteinase-, DNAse-Inhibitoren, etc.

Des weiteren ist Gegenstand der vorliegenden Erfindung ein Arzneimittel, das mindestens zwei verschiedene erfindungsgemäße Strukturproteine enthält, die jeweils unterschiedliche Modifikationen aufweisen. Besonders bevorzugt ist dabei, daß sie unterschiedliche Antigenität besitzen.

Ein weiterer bevorzugter Gegenstand ist ein Kit, enthaltend mindestens zwei verschiedene erfindungsgemäße Strukturproteine, bei dem jedes Strukturprotein getrennt von dem/den anderen Strukturprotein(en) in dem Kit vorliegt.

Für eine Anwendung des Kits bzw. des Arzneimittels mit mindestens zwei verschiedenen erfindungsgemäßen Strukturproteinen, beispielsweise im Rahmen einer Therapie, wird dabei zunächst ein Strukturprotein angewandt. Für eine oder mehrere spätere Anwendung(en) wird/werden Strukturproteine mit anderer Antigenität verwendet. Eine Therapie mit Hilfe des Arzneimittels bzw. Kits umfaßt also die sukzessive Gabe erfindungsgemäßer Strukturproteine. Das Arzneimittel bzw. der Kit haben damit den Vorteil, daß (1) die bei wiederholter Anwendung des gleichen Strukturproteins ausgelöste Potenzierung einer Immunantwort vermieden werden kann und daß (2) im Fall der Auslösung einer Immunreaktion während der ersten Anwendung, durch Verwendung eines Strukturproteins mit unterschiedlicher Antigenität, die vorhandene Abwehrreaktion gegen diese zweite Anwendung weniger wirksam als gegen eine Anwendung mit dem ersten Strukturprotein ausfällt. Die somit verminderte Immunisierung des Patienten erhöht die Wirksamkeit. Für fortlaufende Anwendungen kann so mehrfach zwischen verschiedenen Strukturproteinen gewechselt werden, um eine Immunisierung eines Patienten so niedrig als möglich zu halten. Bevorzugt ist ein Satz von mehreren Strukturproteinen in Form von infektiösen Partikeln mit unterschiedlicher Antigenität, die als Vektor für den mehrfachen Transfer von beispielsweise identischen therapeutischen Genen verwendet werden. Ein weiteres Arzneimittel umfaßt einen Satz von Strukturproteinen in Form von infektiösen Partikeln, die als Vektor für unterschiedliche Therapien verwendet werden.

Ein weiterer Gegenstand der vorliegenden Erfindung bezieht sich auf die Verwendung des erfindungsgemäßen Strukturproteins für die Änderung der Antigenität von AAV, für die Transformation einer Zelle und/oder - in Form von geeigneten rAAV-Vektoren für die Gentherapie. Unter Gentherapie versteht man eine Therapieform, bei der durch die Einführung von Nukleinsäuren in Zellen ein Effektorgen und somit meist ein Protein exprimiert wird. Man unterscheidet prinzipiell In-vitro- und In-vivo-Verfahren. In In-vitro-Verfahren werden Zellen aus dem Organismus entfernt und ex-vivo mit Vektoren transduziert, um anschließend wieder in denselben oder in einen anderen Organismus eingebracht zu werden. Bei der In-vivo-Gentherapie werden Vektoren, beispielsweise zur Bekämpfung von Tumoren, systemisch (z.B. über die Blutbahn) oder lokal (z.B. in den Tumor) appliziert.

Ein wesentlicher Vorteil der vorliegenden Erfindung ist, daß durch die erfindungsgemäße Mutagenese von Strukturproteinen von AAV die Antigenität im wesentlichen ohne Verlust der Verpackungseffizienz rekombinanter AAV-Vektoren - und damit der Grundvoraussetzung der Infektiosität - innerhalb des Kapsids des Virus geändert werden kann. Die vorliegende Erfindung eignet sich daher im besonderen Maße für die in vivo Transduktion von Zellen, beispielsweise für die somatische Gentherapie, wenn eine verminderte Immunisierung von Patienten erwünscht ist.

Die folgenden Beispiele sollen die Erfindung näher erläutern, ohne sie zu beschränken.

### Beispiel 1

### P1-Mutation im VP3:

Zunächst wurde von einem Plasmid pUC-AV2, das durch Subklonierung des 4.8-kb BglII-Fragments des pAV2 (ATCC 37261, ref. 53) in die BamHI Schnittstelle des pUC19 (New England BioLabs Inc.) hergestellt wurde, ausgegangen. Mittels dem Fachmann bekannter PCR-unterstützter Mutagenese wurden an definierten Stellen des Plasmids Mutationen vorgenommen. Dabei wurde eine für P1, ein 14-AS-Peptid, mit der AS-Sequenz QAGTFALRGDNPQG, das das RGD-Bindungsmotiv eines Lamininfragments (Aumailley et al. (1990) FEBS Lett. 262, 82-86) enthält, codierende Sequenz nach den Nukleotiden 2985, 3345 und 3963 eingefügt. Dies entspricht einer Insertion nach den Aminosäuren 261, 381 und 587 des AAV2-Kapsidproteins (Nomenklatur nach Zahl der Aminosäuren (AS) gezählt nach den AS ab Beginn des N-Terminus im VP-1 von AAV2). In der anschließenden PCR werden jeweils 2 mutationsspezifische Primer und als Matrize ein Plasmid, pCap verwendet, das nur das cap-Gen enthält und dadurch gebildet wird, daß das 2.2 kb EcoRI-BspMI-Fragment aus pUC-AV2 herausgeschnitten und in die EcoRI-Schnittstelle des pUC19 eingefügt wird. Anschließend werden die PCR Produkte in Bakterien amplifiziert, sequenziert und das 1.4-kb EcoNI-XcmI-Fragment, das P 1 enthält in pUC-AV2, in dem die korrespondierende Wildtypcap-Sequenz herausgeschnitten wurde, subkloniert. Dementsprechend enthielten die nach den AS-Insertionsstellen pI-261, pI-381 und pI-587 genannten Plasmide (Mutanten) das komplette AAV2-Genom. Die entsprechenden Proteine werden mit I-261, I-381 und I-587 bezeichnet.

### Beispiel 2

### Herstellung von AAV2-Partikeln

HeLa-Zellen (eine humane Cervix-Epithel-Zellinie) wurden mit den Plasmiden gemäß Beispiel 1 transfiziert, dann ca. 20h inkubiert und anschließend mit Adenovirus Typ 5 infiziert. 72 h nach der Infektion wurden die Zellen aufgeschlossen und die AAV2-Partikel über einen CsCl-Gradienten gereinigt.

### Beispiel 3

### Charakterisierung der Kapsidmutanten gemäß Beispiel 1

In diesen Versuchen sollte festgestellt werden, ob die Kapsidmutanten das virale Genom verpacken und vollständige Kapside bilden können. AAV2-Partikel der Mutanten gemäß Beispiel 2 wurden darauf überprüft, ob und wenn ja wieviele Partikel das Virus-Genom tragen und wieviel DNA in den Kapsid-Mutanten verpackt war. Dazu wurden die gemäß Beispiel 2 gereinigten Viren (Mutanten und Wildtyp) mit DNAse behandelt, geblottet und mit einer Rep-Sonde hybridisiert.

Der sich daraus ergebende Titer zeigte keine quantitative oder qualitative Differenz im Vergleich zum Wildtyp (s. Tabelle 1). Die Viren behielten die Fähigkeit, das Genom zu verpacken.

Durch Elektronenmikroskopanalyse konnte weiter bestätigt werden, daß auch das Kapsid ausgebildet wird.

Daher wurden die Mutationen nicht in Bereichen vorgenommen, die für die korrekte Faltung, die Kapsid-Zusammensetzung oder die Verpackung des Genoms von Bedeutung sind. Die erfindungsgemäßen AAV-Partikel sind in ihrer Funktion ungestört.

### Beispiel 4

### Antigenität der Kapsidmutanten gemäß Beispiel 1

Um die Antigenität der mutierten Kapside ablesen zu können, wurden in einem weiteren Experiment A20 monoklonale Antikörper (A20MAb) in einem ELISA eingesetzt. A20MAb reagieren spezifisch mit komplett zusammengesetztem AAV2-Kapsid des Wildtyps (Wistuba et al., (1997), J. Virol. 71, 1341-1352). Auch hier sind die Ergebnisse in Tabelle 1 dargestellt. Dabei zeigt sich, daß durch die Insertion in den Mutanten I-261 und I-381 im Gegensatz zum Wildtyp und I-587 der A20 monoklonale Antikörper nicht mehr binden kann.

**Tabelle 1 Verpackungseffizienz und Antigenität der hergestellten Virusmutanten gemäß Beispiel 1**

| Virusstock | genomische Virustiter | ELISA mit A20-MAb |
|---|---|---|
| Wildtyp-Kapsid | 8·10¹³ | 6·10¹² |
| Mutanten | | |
| I-261 | 1·10¹² | n.m. |
| I-381 | 1·10¹² | n.m. |
| I-587 | 4·10¹³ | 3·10¹² |

Gezeigt sind die genomischen Virustiter (Dot-Blot) und der Titer mit A20-Kapsid-ELISA. Die Konzentrationen sind in Partikel/ml angegeben. "n.m." heißt "nicht meßbar".

### Beispiel 5

### Infektionstests mit Kapsidmutanten gemäß Beispiel 1

Um den Tropismus der Kapsidmutanten I-261, I 381 und I-587 zu testen, wurden die Zellen der Zellinie Co-115 mit den mutierten Viren infiziert. Co-115-Zellen wurden zum Testen des Wildtyprezeptor-Tropismus der Virionen verwendet, da diese gegenüber Wildtyp AAV2 anfällig sind. Drei Tage nach der Infektion wurden die Zellen durch Immunofluoreszenzmessung mit Hilfe eines anti-Rep-Antikörpers darauf untersucht, ob das virale Rep-Protein exprimiert wird (Wistuba et al. (1997) J. Virol. 71, 1341-1352; Wistuba et al. (1995) J. Virol. 69, 5311-5319). Zellen wurden auf Objektträgern zu 70 % Konfluenz gezüchtet und mit verschiedenen Konzentrationen erfindungsgemäßer viraler Präparationen in serumfreiem Medium zusammen mit Adenovirus 5 inkubiert. Die Titer der viralen Präparationen wurden drei Tage später entweder durch in-situ-Detektion der Rep-Proteinsynthese in einem Immunofluoreszenzassay (Rep-Titer) bestimmt. Dabei wurde die Immunofluoreszenzanfärbung mit AAV2-infizierten Zellen nach einer Methode von Wistuba et al. (Wistuba et al. (1997) J. Virol. 71, 1341-1352; Wistuba et al. (1995) J. Virol. 69, 5311-5319) durchgeführt. Die Objektträger wurden einmal mit PBS gewaschen, in Methanol (5 min, 4°C) fixiert und anschließend mit Aceton (5 min, 4°C) behandelt. Die Zellen wurden dann für eine Stunde bei Raumtemperatur mit dem monoklonalen Antikörper 76-3, der mit Rep-Proteinen von AAV2 reagiert, inkubiert. Anschließend wurde gewaschen und für eine Stunde mit einem Rhodamin-konjugierten Anti-Maus-sekundären Antikörper bei einer Verdünnung von 1:50 in PBS mit 1% BSA inkubiert. Die Titer wurden aus der letzten limitierenden Verdünnung der viralen Stammlösung errechnet, die zu fluoreszenzpositiven Zellen geführt hatten.

Nach Infektion mit Wildtyp AAV2 und den Mutanten I-261 und I-587 konnten Rep-positive CO115-Zellen detektiert werden, wobei die Infektiosität der Mutanten um zwei bis drei Größenordnungen kleiner war als die des Wildtyps, bzw. eine Mutante nicht infektiös war (I-381) (Tabelle 2). Es konnte aber gezeigt werden, daß bei der Mutante I-261 trotz verringerer Antigenität (s. Beispiel4) die Infektiosität erhalten blieb.

**Tabelle 2: Virustiter auf der Zelloberfläche**

| Virusstock | Titer auf CO115-Zellen |
|---|---|
| Wildtyp-Kapsid | 2·10⁹ |
| Mutanten | |
| I-261 | 7·10⁶ |
| I-381 | n.m. |
| I-587 | 1·10⁷ |

Gezeigt sind die Titer auf den wildtypanfälligen CO115-Zellen. Die Titer sind für I-261, I-381 und I-587 wie den Wildtyp in Rep-EFU/ml ausgedrückt. Dabei bedeutet EFU expressionsbilende Einheiten (Expressing Forming Unit). Dabei heißt "n.m." "nicht meßbar".

## Patentansprüche

1. Strukturprotein von Adeno-assoziiertem Virus (AAV), **dadurch gekennzeichnet, daß** das Strukturprotein mindestens eine Modifikation enthält, die eine Verringerung der Antigenität des Virus bewirkt.

2. Strukturprotein nach Anspruch 1, **dadurch gekennzeichnet, daß** die Modifikation keine wesentliche Verringerung der Infektiosität des Virus bewirkt.

3. Strukturprotein nach einem der Ansprüche 1 oder 2, **dadurch gekennzeichnet, daß** das mutierte Strukturprotein zur Partikelbildung befähigt ist.

4. Strukturprotein nach einem der Ansprüche 1 bis 3, **dadurch gekennzeichnet, daß** es ausgewählt ist aus modifiziertem VP1, modifiziertem VP2 und/oder modifiziertem VP3.

5. Strukturprotein nach einem der Ansprüche 1 bis 4, **dadurch gekennzeichnet, daß** es abgeleitet ist von AAV1, AAV2, AAV3, AAV4, AAV5 und/oder AAV6 sowie anderen von diesen, insbesondere von AAV2, abgeleiteten AAV-Serotypen.

6. Strukturprotein nach einem der Ansprüche 1 bis 5, **dadurch gekennzeichnet, daß** die Modifikation(en) an der Virusoberfläche lokalisiert ist/sind.

7. Strukturprotein nach einem der Ansprüche 1 bis 6, **dadurch gekennzeichnet, daß** die Modifikation(en) am N-Terminus des Strukturproteins lokalisiert ist/sind.

8. Strukturprotein nach einem der Ansprüche 1 bis 7, **dadurch gekennzeichnet, daß** die Modifikation auf einer kovalenten oder nichtkovalenten Bindung einer oder mehrer hoch- oder niedermolekularen Verbindung, beispielsweise von Biotin, eines Mono- oder Oligosaccharids, einer Hydroxidgruppe oder eines F_{ab}-Fragments, an eine oder mehrerer Aminosäuren oder Aminosäuresequenzen beruht.

9. Strukturprotein nach einem der Ansprüche 1 bis 7, **dadurch gekennzeichnet, daß** die Modifikation eine Mutation ist, beispielsweise eine Punktmutation, eine Mutation mehrerer Aminosäuren, eine oder mehrere Deletion(en), eine oder mehrere Insertion(en) oder eine Kombination dieser Mutationen ist.

10. Strukturprotein nach Anspruch 9, **dadurch gekennzeichnet, daß** Protein oder Peptid, vorzugsweise immunsuppressives Protein oder Peptid insertiert wird.

11. Strukturprotein nach einem der Ansprüche 1 bis 8, **dadurch gekennzeichnet, daß** das Strukturprotein mindestens eine weitere Modifikation enthält.

12. Strukturprotein nach Anspruch 11, **dadurch gekennzeichnet, daß** die weitere(n) Modifikation(en) eine Änderung der Infektiosität des Virus bewirkt.

13. Strukturprotein nach einem der Ansprüche 11 oder 12, **dadurch gekennzeichnet, daß** die weitere(n) Modifikation(en) eine oder mehrere Deletion(en), eine oder mehrere Insertion(en) oder eine Kombination dieser Modifikationen ist.

14. Strukturprotein nach einem der Ansprüche 11 bis 13, **dadurch gekennzeichnet, daß** die Insertion ein Zellmembranrezeptor-Ligand, ein Rep-Protein oder -Peptid, ein immunsuppressives Protein oder Peptid und/oder ein Protein oder Peptid mit einem Signal zur Doppelstrangsynthese des Fremdgens ist.

15. Strukturprotein nach einem der Ansprüche 10 bis 14, **dadurch gekennzeichnet, daß** die Insertion ausgewählt ist aus einem Integrin, einem Cytokin oder einer Rezeptor-Bindungsdomäne von einem Cytokin,-Integrin oder Wachstumsfaktor, an einem Zelloberflächenrezeptor bindenden einzelkettigen Antikörper, einem Antikörper gegen Zelloberflächenstrukturen, einer antikörperbindenden Struktur oder einem Epitop.

16. Strukturprotein nach einem der Ansprüche 1 bis 15, **dadurch gekennzeichnet, daß** die Modifikation(en) durch eine oder mehrere Insertionen an der XhoI-Schnittstelle der VP1-kodierenden Nukleinsäure bewirkt wird/werden.

17. Strukturprotein nach einem der Ansprüche 1 bis 16, **dadurch gekennzeichnet, daß** die Modifikation(en) durch eine oder mehrere Insertionen an der BsrBI-Schnittstelle der VP1-kodierenden Nukleinsäure bewirkt wird/werden.

18. Strukturprotein nach einem der Ansprüche 1 bis 17, **dadurch gekennzeichnet, daß** die Modifikation(en) durch eine oder mehrere Deletionen zwischen den BsrBI-HindII-Schnittstellen der VP1-kodierenden Nukleinsäure und eine oder mehrere Insertionen bewirkt wird/werden.

19. Strukturprotein nach einem der Ansprüche 1 bis 18, **dadurch gekennzeichnet, daß** die Modifikation(en) durch eine oder mehrere Deletionen zwischen den XhoI-XhoI-Schnittstellen der VP1-kodierenden Nukleinsäure bewirkt wird/werden.

20. Strukturprotein einem der Ansprüche 1 bis 19, **dadurch gekennzeichnet, daß** die Modifikation(en) durch eine oder mehrere Deletionen zwischen den BsrBI-HindII-Schnittstellen der VP1-kodierenden Nukleinsäure bewirkt wird/werden.

21. Strukturprotein nach einem der Ansprüche 1 bis 15, **dadurch gekennzeichnet, daß** eine oder mehrere Insertionen in VP3 vor und/oder nach mindestens einer Aminosäure in der Sequenz ausgewählt aus YKQIS SQSGA, YLTLN NGSQA, YYLSR TNTPS, EEKFF PQSGV, NPVAT EQYGS, LQRGN RQAAT, NVDFT VDTNG, lokalisiert ist/sind.

22. Strukturprotein gemäß einem der Ansprüche 1 bis 21 in Form eines AAV-Partikels, insbesondere in Form eines AAV-Kapsids.

23. Nukleinsäure, kodierend für ein Strukturprotein gemäß einem der Ansprüche 1 bis 22.

24. Zelle, enthaltend eine Nukleinsäure gemäß Anspruch 22.

25. Verfahren zur Herstellung eines Strukturproteins gemäß einem der Ansprüche 1 bis 21, **dadurch gekennzeichnet, daß** eine Zelle gemäß Anspruch 23 kultiviert und ggf. das exprimierte Strukturprotein isoliert wird.

26. Arzneimittel, enthaltend ein Strukturprotein gemäß einem der Ansprüche 1 bis 21, eine Nukleinsäure gemäß Anspruch 22 und/oder eine Zelle gemäß Anspruch 23 und/oder gegebenenfalls Hilfs- und/oder Zusatzstoffe.

27. Arzneimittel enthaltend mindestens zwei verschiedene Strukturproteine gemäß einem der Ansprüche 1 bis 21, **dadurch gekennzeichnet, daß** jedes Strukturprotein eine unterschiedliche Modifikation aufweist.

28. Kit enthaltend mindestens zwei verschiedene Strukturproteine gemäß einem der Ansprüche 1 bis 21, **dadurch gekennzeichnet, daß** jedes Strukturprotein getrennt von dem/den anderen Strukturprotein(en) in dem Kit vorliegt.

29. Verwendung eines Strukturproteins gemäß einem der Ansprüche 1 bis 21, einer Nukleinsäure gemäß Anspruch 22 und/oder einer Zelle gemäß Anspruch 23 für die Änderung der Antigenität von AAV, für die Transformation einer Zelle und/oder für die Gentherapie.
